# EUROPÄISCHE PATENTSCHRIFT

(11) **EP 0 873 736 B1**
(45) Veröffentlichungstag und Bekanntmachung des Hinweises auf die Patenterteilung: **24.09.2003**
(21) Anmeldenummer: 98107123.6
(22) Anmeldetag: 20.04.1998
(51) Int. Cl.: A61F 13/10, A61F 5/30

(54) **Epikondylitis-Spange**
Epikondylitis brace
Bandage d'épicondylitis

(30) Priorität: 21.04.1997 DE 19716705
(43) Veröffentlichungstag der Anmeldung: 28.10.1998
(73) Patentinhaber: Bauerfeind Orthopädie GmbH & Co. KG, 47906 Kempen (DE)
(72) Erfinder: Reinhardt, Holger, 47906 Kempen (DE); Bauerfeind, Hans Bruno, 47906 Kempen (DE)
(74) Vertreter: Bardehle, Heinz, Dipl.-Ing.

(56) Entgegenhaltungen:
- DE-A- 2 635 426
- DE-A- 4 336 545
- FR-A- 2 596 980
- US-A- 4 299 214
- US-A- 5 295 951

## Beschreibung

Die Erfindung bezieht sich auf eine Epikondylitis-Spange mit einer ein Federband enthaltenden Klammer, die durch ein stufenlos verstellbares Halteband zusammenziehbar ist.

Eine derartige Epikondylitis-Spange ist aus der DE 26 35 426 C2 bekannt, die eine Klammer mit einer etwa halbringförmigen Feder enthält und an ihrem einen Ende eine Druckplatte aufweist, die sich beim Zusammenziehen der Klammer mittels eines mit einem Klettverschluß versehenen Haltebandes an den Unterarm andrückt, ohne dabei jedoch in diesem Bereich einen besonderen Druck auszuüben, da die Spange kein Polster oder eine Pelotte aufweist.

Eine andere Epikondylitis-Spange ist aus dem DE 9115993 U1 bekannt. Diese Spange enthält eine bogenförmige Klammer, deren Enden mittels eines Haltebandes zusammenziehbar sind, wobei sich die Klammer um den betreffenden Bereich des Unterarms zusammenzieht. Eine auf der Innenseite der Klammer angebrachte Pelotte drückt dabei auf den von ihr beaufschlagten Teil des Unterarms und soll damit seine therapeutische Wirkung entfalten.

Schließlich sei noch auf das DE 9316368 U1 verwiesen, in dem eine Epikondylitis-Spange offenbart ist, die im wesentlichen aus einer bandförmigen Bandage besteht, auf deren Innenseite zwei verschiebbare Druckpolster angebracht sind. Die Bandage läßt sich mittels eines Haltebandes zusammenziehen, wobei durch die an die gewünschte Stelle geschobenen Polster ein Druck auf den Epikondylus im Sinne einer therapeutischen Beeinflussung ausgeübt wird.

Der Erfindung liegt die Aufgabe zugrunde, eine Epikondylitis-Spange zu schaffen, mit der sich eine einstellbare Druckeinleitung auf den Epikondylus in therapeutisch besonders günstiger Weise unabhängig davon erzielen läßt, ob die Spange am rechten oder linken Arm angelegt wird.

Erfindungsgemäß geschieht dies dadurch, daß an dem einen Ende der Klammer an der Innenseite der Spange eine Pelotte zur Druckausübung auf den Epikondylus angeordnet ist, die als längliches, dreiecksähnliches Polster mit quer zur Umfassungsrichtung der Klammer liegender Längsrichtung und einseitiger Verbreiterung gestaltet und über eine mittige, senkrecht zur Längsrichtung der Pelotte liegenden Achse derart drehbar an der Spange befestigt ist, daß je nach Anlegen am rechten oder linken Arm die Verbreiterung zum Oberarm hinweist.

Die an dem einen Ende der Klammer angeordnete Pelotte in Form des länglichen, dreiecksähnlichen Polsters hat sich als besonders intensiv wirkende Gestaltung erwiesen, die die anatomischen Verhältnisse am Unterarm berücksichtigt und die auf der Außenseite des Unterarms in Richtung zur Elle eine therapeutisch erwünschte breitere Druckfläche gegenüber einer schmäleren Druckfläche in Richtung weg von der Elle schafft. Die Drehbarkeit der Pelotte ermöglicht es dabei, sie jeweils quer zur Längsrichtung der Klammer so einzustellen, daß die verbreiterte Seite an dem einen Ende der Pelotte entweder auf der linken oder der rechten Seite der Klammer liegt, und zwar bei Blickrichtung auf die Innenseite der Klammer. Die Epikondylitis-Spange ist somit universell am rechten oder linken Unterarm und mit hohem therapeutischen Effekt einsetzbar.

Um die von dieser Epikondylitis-Spange ausgehende Druckwirkung besonders auf den Bereich der Pelotte zu konzentrieren, gestaltet man ihre Klammer zweckmäßig so, daß das die Pelotte tragende Ende der Klammer mit deren weiterführendem Bereich über eine Schwächung des Federbandes verbunden ist, die bei Anlegen der Spange und Anziehen des Haltebandes sich scharnierartig biegt und die Pelotte an den Arm drückt. Die Schwächung des Federbandes gibt diesem an der Stelle der Schwächung eine gewisse Scharnierwirkung, so daß durch das Anziehen des Haltebandes zwar die Klammer als Ganzes zusammengezogen wird, jedoch wegen der Scharnierwirkung das die Pelotte tragende Ende der Klammer an den Unterarm besonders herangezogen wird und damit die Druckeinwirkung in diesem Bereich erhöht.

Das Halteband wird zweckmäßig im Bereich der Schwächung an der Klammer über ein Umlenkelement angelenkt, das an der Klammer über ein Elastikband befestigt ist, wobei das Elastikband bei seiner Dehnung die dabei entstehende Spannung über das Umlenkelement in den Bereich der Pelotte einleitet. Aufgrund dieser Anordnung des Umlenkelements und des Elastikbandes ergibt sich eine besonders günstige Einleitung des von der Epikondylitis-Spange ausgeübten Drucks.

Um beim Anlegen der Epikondylitis-Spange die von ihm ausgehende Spannung sichtbar werden zu lassen, führt man das Elastikband zweckmäßig in einer an der Klammer angebrachten Tasche und versieht das Elastikband mit Markierungen, deren Sichtbarwerden bei Dehnung des Elastikbandes dessen Spannung anzeigt.

Die oben erwähnte Schwächung des in der Klammer enthaltenen Federbandes kann man weiterhin dazu ausnutzen, dem die Pelotte tragenden Ende der Klammer eine Verwindungsmöglichkeit gegenüber dem weiteren Bereich der Klammer zu ermöglichen. Eine Verwindung des Bereichs der Klammer mit der Pelotte ist aufgrund der Anatomie des Unterarmes besonders wünschenswert. Um die therapeutische Wirkung des die Pelotte bildenden Polsters zu intensivieren, versieht man diese vorteilhaft an ihren drei Ecken jeweils mit einer Erhebung, die dann in ihrem Bereich einen besonders konzentrierten Druck ausübt. Diese Druckausübung läßt sich dadurch weiterhin intensivieren, daß man die Erhebungen mit einer profilierten Oberfläche versieht.

An dem die Pelotte tragenden Ende der Klammer kann man vorteilhaft ein Sichtfenster vorsehen, das eine Markierung auf der Pelotte sichtbar macht, mit der in Abhängigkeit von deren Verdrehung angezeigt wird, ob die Pelotte zur Anlage an einen rechten oder linken Arm eingestellt ist.

In den Figuren ist ein Ausführungsbeispiel der Erfindung dargestellt. Es zeigen
- Figur 1: die Epikondylitis-Spange in perspektivischer Sicht mit die Klammer zusammenziehenden Halteband,
- Figur 2: die Epikondylitis-Spange gemäß Figur 1 in axialer Sicht und in einem Schnitt längs der Linie A - A aus Figur 4,
- Figur 3: die Epikondylitis-Spange in offener Lage und gestreckter Abwicklung,
- Figur 4: eine Innenansicht der Epikondylitis-Spange im Bereich der Pelotte.

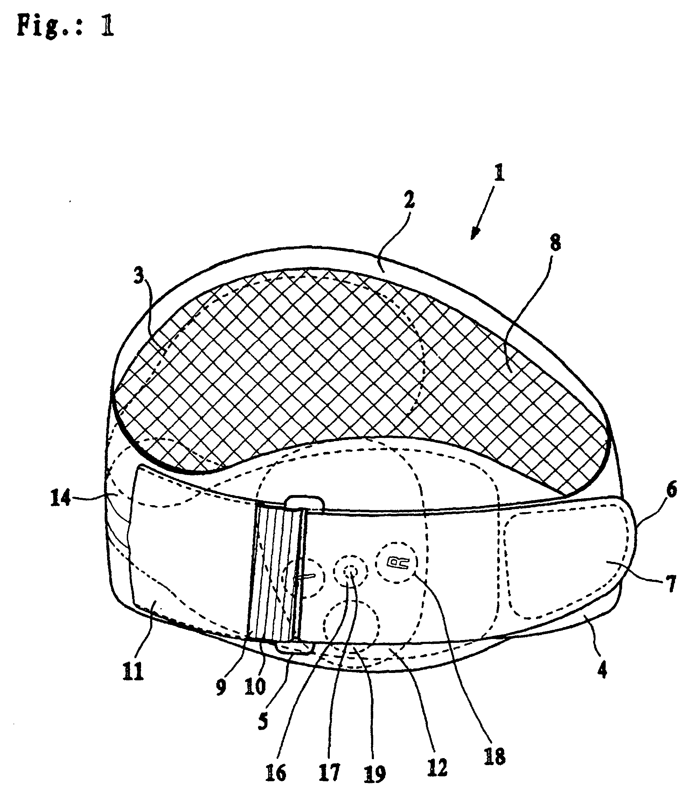

Figur 1 zeigt die Epikondylitis-Spange 1, die aus der Klammer 2 mit dem darin enthaltenen Federband 3 besteht. Das Federband 3 ist in der weiter unten beschriebenen Weise in die Klammer 2 eingeklebt. Die Klammer 2 geht in das gegenüber der Klammer 2 schmalere Halteband 4 über, das in der geschlossen gezeigten Darstellung gemäß Figur 1 durch das Umlenkelement 5 in Form einer gestreckten Öse geschlungen ist und mit seinem Ende 6 über den Anfang des Haltebandes 4 gelegt ist, wo es mittels des Klettverschlusses 7 an dem Halteband 4 festgehalten wird. Die Klammer 2 ist auf ihrer Innenseite mit einer das Tragen der Epikondylitis-Spange erleichternden Textilauflage 8 (kreuzschraffiert) versehen.

Das Umlenkelement 5 ist an der Klammer 2 über das Elastikband 9 befestigt, das aus den weiter unten dargestellten Gründen mit mehreren Quernähten 10 versehen ist. Das Elastikband 9 verläuft auf seiner dem Umlenkelement abgewandten Seite in der auf die Klammer 2 aufgebrachten Tasche 11, in der es an der Klammer 2 befestigt ist. Die Klammer 2 umhüllt das in ihr enthaltene Federband 3 mit einem Überzug aus Textilmaterial, der in das Halteband 4 übergeht und für den Klettverschluß 7 mit seinen Klettelementen das zugehörige Flauschteil bildet.

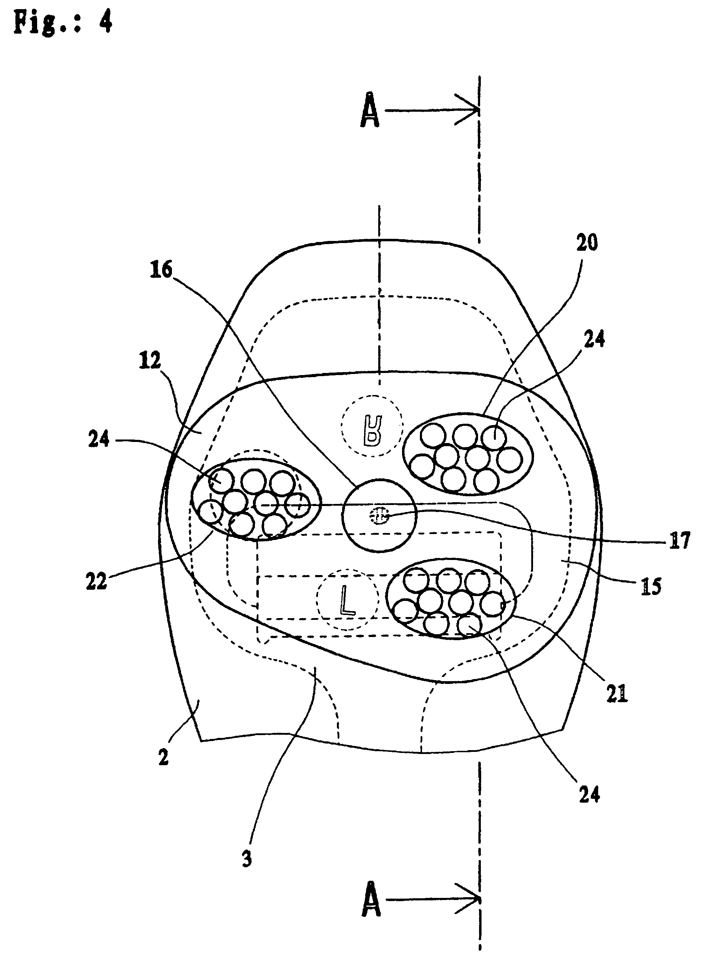

Figur 1 zeigt weiterhin eine gestrichelt gezeichnete, als Polster 12 ausgebildete Pelotte, die in der geschlossen gezeichneten Lage der Epikondylitis-Spange 1 auf der Innenseite der Klammer 2 angeordnet und daher in Figur 1 nicht sichtbar dargestellt ist. Auf das Polster wird weiter unten im Zusammenhang mit der Figur 4 näher eingegangen.

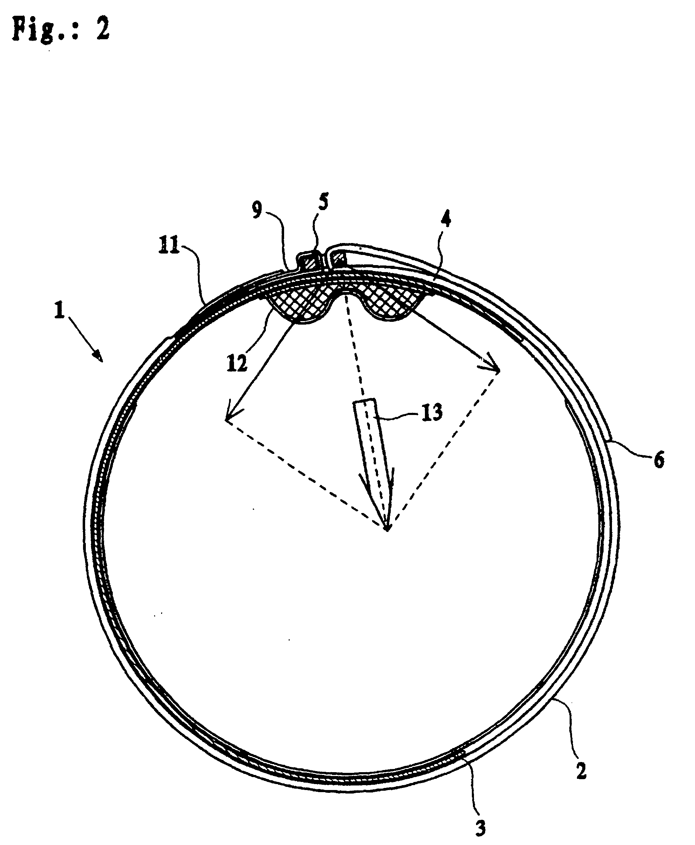

Figur 2 zeigt die in Figur 1 dargestellte Epikondylitis-Spange 1 in einer axialen Sicht, und zwar in einem Schnitt längs der Linie A - A aus Figur 4. Aus Figur 2 wird die Festhaltung des Endes 6 durch das Halteband 4 deutlich, das um das Umlenkelement 5 geschlungen ist und mit seinem Ende 6 gegen den betreffenden Teil des Haltebandes 4 anliegt, wo es durch die Wirkung des in Figur 1 dargestellten Klettverschlusses 7 festgehalten wird. In die Klammer 2 ist das Federband 3 eingearbeitet, das sich über den größten Teil der Klammer 2 erstreckt und dieser eine gewünschte biegeelastische Steifigkeit gibt.

Figur 2 zeigt weiterhin die Befestigung des Umlenkelements 5 über das Elastikband 9 in der Tasche 11, aus der das Elastikband 9 über ein kurzes Stück herausragt. Unter dem Einfluß des von dem umgelegten Ende 6 des Haltebandes 4 auf das Umlenkelement ausgeübten Zuges ergibt sich entsprechend dem in Figur 2 eingezeichneten Parallelogramm der Kräfte eine durch den Pfeil 13 dargestellt Kraft, die auf denjenigen Unterarmteil wirkt, der dem Polster 12 gegenüberliegt. Um dabei den gewünschten therapeutischen Effekt zu erzielen, wird die dargestellte Epikondylitis-Spange 1 so am Unterarm angelegt, daß das Polster 12 in der vom behandelnden Arzt gewünschten Lage in bezug auf den Epikondylus angeordnet ist.

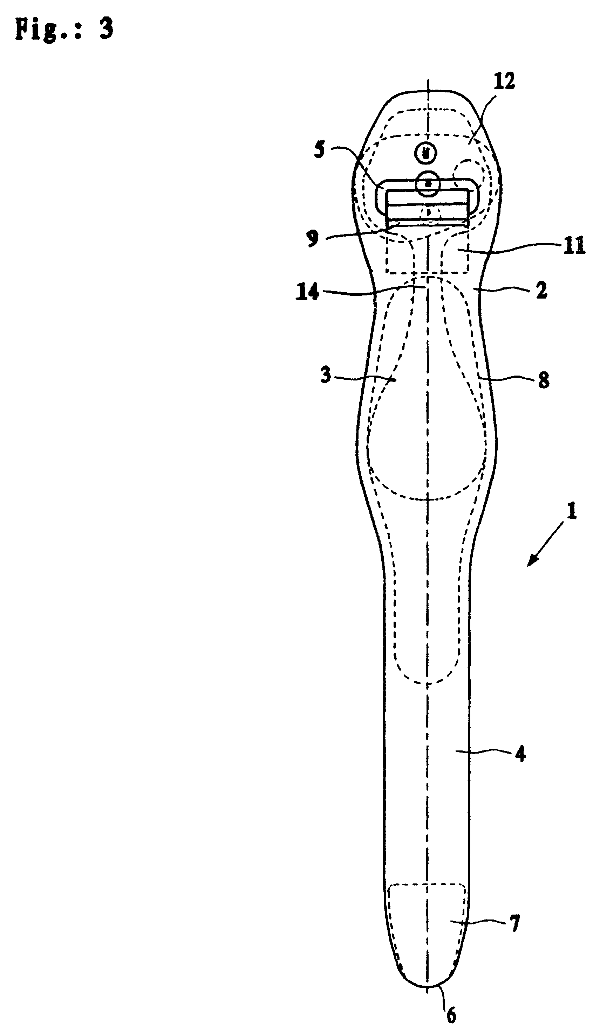

Figur 3 zeigt die in den Figuren 1 und 2 dargestellte Epikondylitis-Spange 1 in gestreckter abgewickelter Lage, und zwar in Draufsicht auf das von außen ersichtliche Umlenkelement 5 mit dem gestrichelt gezeichneten Federband 3, das hier die Schwächung 14 zeigt, durch die das Federband 3 gegenüber seinen Endbereichen erheblich verschmälert ist. Aufgrund dieser Schwächung 14 des Federbandes 3 gibt diese dem Federband 3 eine gewisse Scharnierwirkung im Bereich der Schwächung 14, so daß beim straffen Anziehen des Haltebandes 4 der das Polster 12 tragende Teil der Klammer 2 sich gegenüber der sonstigen Rundung der Klammer 2 nach innen zu abbiegt und damit in der Lage ist, in der in Figur 2 dargestellten Richtung des Pfeiles 13 einen besonderen Druck auf den an dieser Stelle liegenden Teil des Unterarmes auszuüben. Weiterhin gibt die Schwächung 14 der Klammer 2 die Möglichkeit, sich mit ihrem das Polster 12 tragenden Teil gegenüber dem weiteren Bereich der Klammer 2 zu verwinden, um sich damit der Form des Unterarmes bequem anpassen zu können.

Der beim Umlegen des Endes 6 des Haltebandes 4 entstehende Zug auf das Umlenkelement 5 bewirkt, daß das Elastikband 9, von dem das Halteband 9 gehalten wird, aufgrund seiner Elastizität aus der Tasche 11 entsprechend dem vom Halteband 4 ausgeübten Zug herausgezogen wird, wobei mehr oder weniger Quernähte 10 (siehe Figur 1) in Erscheinung treten, anhand deren Zahl damit die betreffende Zugkraft und über diese der auf den Unterarm ausgeübte Druck abgelesen werden kann. Anstelle der Quernähte 10 können natürlich auch verschiedene Farbstreifen verwendet werden.

In der Figur 4 ist das das Polster 12 tragende Ende der Klammer 2 dargestellt, und zwar in einer Sicht von innen her, so daß das Polster 12 direkt sichtbar ist. Das Polster 12 besitzt eine Umfassung ähnlich einem Dreieck mit der einseitigen Verbreiterung 15, wobei das Polster 12 in seiner durch die Dreiecksform gegebenen Längserstreckung quer zur Längsrichtung der Klammer 2 angeordnet ist. Das Polster 12 ist über die durch den Niet 16 gebildete Achse 17 drehbar an der Klammer 2 gehalten, wobei der Niet 16 dafür sorgt, daß das Polster 12 satt an der Klammer 2 unter Erhaltung seiner Verdrehbarkeit anliegt. Das Polster 12 weist auf seiner der Klammer 2 zugewandten Seite die beiden Markierungen "R" und "L" auf, die die Zugehörigkeit zum rechten oder linken Arm bezeichnen. Diese Markierungen sind durch ein Fenster 18 (siehe Fig. 1) in der Klammer 2 zu sehen, so daß der Benutzer der Epikondylitis-Spange je nach Verdrehung des Polsters 12 sofort erkennt, ob die Spange für den rechten oder den linken Arm eingestellt ist. In der Figur 1 ist zusätzlich durch den gestrichelt gezeichneten Kreis 19 eine Haftklebestelle angedeutet, über die das Polster 12 in der jeweils eingestellten Lage gehalten wird.

Aus der in Figur 4 dargestellen Ausbildung des Polsters 12 ist ersichtlich, daß dieses an seinen durch die ungefähre Dreiecksform gebildeten Ecken jeweils mit einer Erhebung 20, 21 und 22 versehen ist, die einen besonderen therapeutischen Effekt infolge der Ausübung eines erhöhten Druckes an den betreffenden Stellen bewirkt. Darüber hinaus sind die drei Erhebungen 20, 21 und 22 mit Noppen 24 versehen, durch die als profilierte Oberfläche ebenfalls eine besondere therapeutische Wirkung erzielt wird. Es sei jedoch darauf hingewiesen, daß das Polster auch mit einer um den Niet 16 herum geformten durchgehenden einfachen Wölbung versehen sein kann.

## Patentansprüche

1. Epikondylitis-Spange (1) mit einer ein Federband (3) enthaltenden Klammer (2), die durch ein stufenlos verstellbares Halteband (4) zusammenziehbar ist, **dadurch gekennzeichnet, daß** an dem einen Ende der Klammer (2) an der Innenseite der Spange (1) eine Pelotte (12) zur Druckausübung auf den Epikondylus angeordnet ist, die als längliches, dreiecksähnliches Polster mit quer zur Umfassungsrichtung der Klammer (2) liegender Längsrichtung und einseitiger Verbreiterung (15) gestaltet und über eine mittige, senkrecht zur Längsrichtung der Pelotte liegenden Achse (17) derart drehbar an der Spange (1) befestigt ist, daß je nach Anlegen am rechten oder linken Arm die Verbreiterung (15) zum Oberarm hinweist.

2. Spange nach Anspruch 1, **dadurch gekennzeichnet, daß** das die Pelotte (12) tragende Ende der Klammer (2) mit deren weiterführenden Bereich über eine Schwächung (14) des Federbandes (3) verbunden ist, die bei Anlegen der Spange (1) und Anziehen des Haltebandes (4) sich scharnierartig biegt und die Pelotte (12) an den Arm drückt.

3. Spange nach Anspruch 2, **dadurch gekennzeichnet, daß** das Halteband (4) im Bereich der Schwächung (14) an der Klammer (2) über ein Umlenkelement (5) angelenkt ist, das an der Klammer (2) über ein Elastikband (9) befestigt ist, wobei das Elastikband (9) bei seiner Dehnung die dabei entstehende Spannung (13) das Umlenkelement (5) in den Bereich der Pelotte (12) einleitet.

4. Spange nach Anspruch 3, **dadurch gekennzeichnet, daß** das Elastikband (9) in einer an der Klammer (2) angebrachten Tasche (11) geführt und mit Markierungen (10) versehen ist, deren Sichtbarwerden bei Dehnung des Elastikbandes (9) dessen Spannung anzeigt.

5. Spange nach einem der Ansprüche 2 bis 4, **dadurch gekennzeichnet, daß** die Schwächung (14) eine Verwindung des die Pelotte (12) tragenden Endes gegenüber dem weiterführenden Bereich der Klammer (2) ermöglicht.

6. Spange nach einem der Ansprüche 1 bis 5, **dadurch gekennzeichnet, daß** die Pelotte (12) an ihren drei Ecken jeweils eine Erhebung (20, 21, 22) aufweist.

7. Spange nach Anspruch 6, **dadurch gekennzeichnet, daß** die Erhebungen (20, 21, 22) mit einer profilierten Oberfläche (24) versehen sind.

8. Spange nach einem der Ansprüche 1 bis 7, **dadurch gekennzeichnet, daß** an dem die Pelotte (12) tragenden Ende der Klammer (2) ein Sichtfenster (18) vorgesehen ist, das eine Markierung (R, L) auf der Pelotte (12) in Abhängigkeit von deren Verdrehung sichtbar macht.

## Claims

1. An epicondylitis clasp (1) having a brace (2) which contains a spring strip (3) and can be tightened by an infinitely adjustable retaining strap (4), wherein arranged at one end of the brace (2) on the inside of the clasp (1) is a pad (12) which serves to exert pressure on the epicondyle, is configured as an elongated, roughly rectangular cushion, having a longitudinal direction transverse to the clasping direction of the brace (2) and a widened part (15) at one end, and is fastened rotatively to the clasp (1) via a central axis (17) perpendicular to the longitudinal direction of the pad in such a way that the widened part (15) points toward the upper arm, whether applied to the right or left arm.

2. The clasp as claimed in claim 1, wherein the end of the brace (2) bearing the pad (12) is joined to the further region of said brace via a weakened part (14) of the spring strip (3) which bends like a hinge when the clasp (1) is applied and the retaining strap (4) is tightened, and presses the pad (12) against the arm.

3. The clasp as claimed in claim 2, wherein in the region of the weakened part (14) the retaining strap (4) is hinged to the brace (2) via a deflecting element (5) which is fastened to the brace (2) via an elastic band (9), it being the case that as it expands the elastic band (9) introduces the tension (13) thereby produced into the region of the pad (12) via the deflecting element (5).

4. The clasp as claimed in claim 3, wherein the elastic band (9) is guided in a pocket (11) provided on the brace (2), and is provided with markings (10) which, by becoming visible, indicate the tension of the elastic band (9) as it expands.

5. The clasp as claimed in one of claims 2 to 4, wherein the weakened part (14) permits the end bearing the pad (12) to twist with respect to the further region of the brace (2).

6. The clasp as claimed in one of claims 1 to 5, wherein the pad (12) has a raised portion (20, 21, 22) at each of its three corners.

7. The clasp as claimed in claim 6, wherein the raised portions (20, 21, 22) are provided with a profiled surface (24).

8. The clasp as claimed in one of claims 1 to 7, wherein there is provided on the end of the brace (2) bearing the pad (12) a viewing window (18) which makes a marking (R, L) on the pad (12) visible as a function of its torsion.

## Revendications

1. Bracelet épicondylien (1) avec une sangle (2) qui renferme une bande à ressort (3) et qui peut être serrée par l'intermédiaire d'une bande de maintien à réglage progressif (4), **caractérisé en ce qu'**à l'une des extrémités de la sangle (2) est disposée, sur la face intérieure du bracelet (1), une pelote (12) pour exercer une pression sur l'épicondyle, laquelle est conçue sous la forme d'un coussinet allongé semblable à un triangle avec une direction longitudinale orientée transversalement à la direction d'enveloppement de la sangle (2) et avec une partie élargie d'un côté (15) et, par l'intermédiaire d'un axe médian (17) orienté verticalement par rapport à la direction longitudinale de la pelote, est fixée au bracelet (1) de manière tournante, de façon qu'après application sur le bras droit ou sur le bras gauche la partie élargie (15) soit dirigée vers le bras.

2. Bracelet selon la revendication 1, **caractérisé en ce que** l'extrêmité de la sangle (2) portant la pelote (12) est reliée à la zone consécutive par l'intermédiaire d'une partie resserrée (14) de la bande à ressort (3), laquelle, lors de l'application du bracelet (1) et du serrage de la bande de maintien (4) , se plie à la façon dune charnière et presse la pelote (12) contre le bras.

3. Bracelet selon la revendication 2, **caractérisé en ce que**, dans la zone de la partie resserrée (14), la bande de maintien (4) est articulée avec la sangle (2) par l'intermédiaire d'un élément de retournement (5) qui est fixé à la sangle (2) par l'intermédiaire d'une bande élastique (9), la bande élastique (9) transmettant, lors de son extension, la tension ainsi engendrée (13) à la zone de la pelote (12) par l'intermédiaire de l'élément de retournement (5).

4. Bracelet selon la revendication 3, **caractérisé en ce que** la bande élastique (9) est guidée dans une poche (11) disposée sur la sangle (2) et est pourvue de repères (10) dont l'apparition lors de l'extension de la bande élastique (9) indique la tension de celle-ci.

5. Bracelet selon une des revendications 2 à 4, **caractérisé en ce que** la partie resserrée (14) permet de tordre l'extrémité portant la pelote (12) par rapport à la zone consécutive de la sangle (2).

6. Bracelet selon une des revendications 1 à 5, **caractérisé en ce que** la pelote (12) comporte un relief (20, 21, 22) dans chacun de ses trois coins.

7. Bracelet selon la revendication 6, **caractérisé en ce que** les reliefs (20, 21, 22) sont munis d'une surface profilée (24).

8. Bracelet selon une des revendications 1 à 7, **caractérisé en ce qu'**à l'extrémité de la sangle (2) portant la pelote (12) est prévue une fenêtre (18) qui permet de visualiser un repère (R, L) apparaissant sur la pelote (12) en fonction de la torsion de celle-ci.
